(19) Europäisches Patentamt
European Patent Office
Office européen des brevets

(11) Veröffentlichungsnummer : **0 184 731**
**B1**

(12) # EUROPÄISCHE PATENTSCHRIFT

(45) Veröffentlichungstag der Patentschrift : **25.05.88**

(21) Anmeldenummer : **85115154.8**

(22) Anmeldetag : **29.11.85**

(51) Int. Cl.⁴ : **C 07 C 69/732**, C 07 C 93/193, C 07 C121/34, C 07 C 67/343, C 07 C120/00

(54) **Verfahren zur Herstellung von 2-(1-Hydroxymethyl)-acrylnitril und -acrylestern.**

(30) Priorität : **04.12.84 DE 3444098**

(43) Veröffentlichungstag der Anmeldung : **18.06.86 Patentblatt 86/25**

(45) Bekanntmachung des Hinweises auf die Patenterteilung : **25.05.88 Patentblatt 88/21**

(84) Benannte Vertragsstaaten : **CH DE FR GB IT LI**

(56) Entgegenhaltungen :
**FR-A- 1 506 132**
**FR-A- 2 120 686**
**US-A- 2 601 659**

(73) Patentinhaber : **BASF Aktiengesellschaft**
**Carl-Bosch-Strasse 38**
**D-6700 Ludwigshafen (DE)**

(72) Erfinder : **Fikentscher, Rolf, Dr.**
**Von-Stephan-Strasse 27**
**D-6700 Ludwigshafen (DE)**
Erfinder : **Hahn, Erwin, Dr.**
**Am Buechsenackerhang 31**
**D-6900 Heidelberg (DE)**
Erfinder : **Kud, Alexander, Dr.**
**Gartenstrasse 5a**
**D-6753 Enkenbach-Alsenborn (DE)**
Erfinder : **Oftring, Alfred, Dr.**
**Berner Weg 26**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 184 731 B1

**Beschreibung**

Aus der US-PS 3 743 669 ist die Herstellung von 2-(1-Hydroxyalkyl)-acrylnitrilen und von 2-(1-Hydroxyalkyl)-acrylestern der allgemeinen Formel

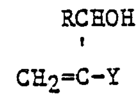

(III)

bekannt, in der Y u.a. = —CN oder

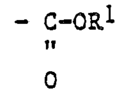

und R un $R^1$ eine Alkyl- oder Arylgruppe bedeuten. Die Verbindungen der Formel III werden hergestellt, indem man Acrylnitril oder einen Acrylester der Formel $CH_2$=CH—Y mit einen Aldehyd der Formel RCHO, wobei die Substituenten Y und R die in Formel III angegebene Bedeutung haben, in Gegenwart von tertiären Aminen als Katalysator bei Temperaturen von 0 bis 200 °C reagieren läßt.

Nach der Lehre der US-PS 2 601 650 erhält man 2-(1-Hydroxymethyl)-acrylnitrile durch Umsetzung von Verbindungen der Formel

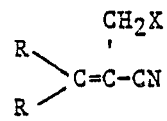

(IV)

mit Basen in wäßrigen Medium. Die Substituenten R in Formel IV bedeuten H oder eine Alkylgruppe, die bis zu 3 Kohlenstoffatome aufweisen kann, der Substituent X steht für Halogen.

2-(1-Hydroxymethyl)-acrylester werden nach dem in der US-PS 3 066 165 beschriebenen Verfahren durch Umsetzung von Propargylalkohol mit Kohlenmonoxid in Gegenwart von Nickeltetracarbonyl und Essigsäure in wäßrigem Medium zu 2-(1-Hydroxymethyl)-acrylsäure und anschließende Veresterung mit einem 1 bis 8 Kohlenstoffatome aufweisenden Alkohol hergestellt.

Die oben beschriebenen Verfahren zur Herstellung von 2-(1-Hydroxylmethyl)-acrylnitril bzw.-acrylestern sind z.T. relativ umständlich und entweder von den Ausgangsmaterialien oder vom Verfahren her gesehen aufwendig oder sie liefern die gewünschten Hydroxymethylverbindungen nur in schlechter Ausbeute.

Aufgabe der Erfindung ist es, ein verbessertes Verfahren zur Herstellung von 2-(1-Hydroxymethyl)-acrylestern zur Verfügung zu stellen.

Die Aufgabe wird erfindungsgemäß mit einem Verfahren zur Herstellung von 2-(1-Hydroxymethyl)-acrylnitril und -acrylestern der Formel

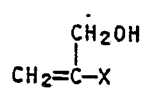

(I)

in der

X = —CN,

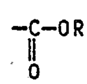 ,

R = $C_1$- bis $C_{18}$-Alkyl, —$(CH_2)_n$—OH,

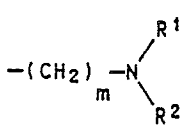 ,

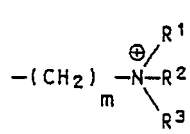

$Y^{\ominus}$, $-(C_2H_4O)pR^4$
$R^1$, $R^2 = -CH_3$, $-C_2H_5$
$R^3 = -H$, $-CH_3$, $-C_2H_5$,

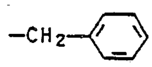

$R^4 = C_1$- bis $C_{18}$-Alkyl,
$n = 2$ bis 4,
$m = 2$ bis 5,
$p = 1$ bis 80 und
$Y^- = Cl^-$, $Br^-$, $SO_4^{2-}$, $PO_4^{3-}$, $CH_3OSO_3^-$, $C_2H_5OSO_3^-$, $CH_3COO^-$, $HCOO^-$
bedeutet, dadurch gelöst, daß man eine Verbindung der Formel

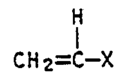 (II)

in der X die in Formel I angegebene Bedeutung hat, mit hydratisiertem Formaldehyd oder Halbacetalen des Formaldehyds, die sich von $C_1$- bis $C_6$-Alkoholen ableiten, in Gegenwart von tertiären Aminen als Katalysator umsetzt.

Als Verbindungen der Formel II kommen Acrylnitril und Acrylester in Betracht. Der Substituent X in Formel II kann folgende Bedeutung haben :

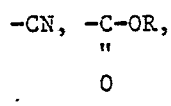

$R = C_1$- bis $C_{18}$-Alkyl,

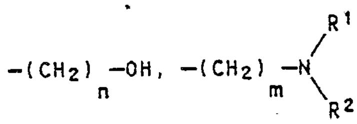

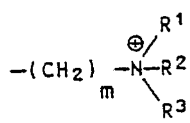

$Y^{\ominus}$, $-(C_2H_4O)_pR^4$
$R^1$, $R^2 = -CH_3$, $-C_2H_5$
$R^3 = -H$, $-CH_3$, $-C_2H_5$,

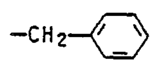

$R^4 = C_1$- bis $C_{18}$-Alkyl,
$n = 2$ bis 4,
$m = 2$ bis 5,
$p = 1$ bis 80 und
$Y^- = Cl^-$, $Br^-$, $SO_4^{2-}$, $PO_4^{3-}$, $CH_3OSO_3^-$, $C_2H_5OSO_3^-$, $CH_3COO^-$, $HCOO^-$.

$C_1$- bis $C_{18}$-Alkylacrylate sind beispielsweise Methylacrylat, Ethylacrylat, n-Propylacrylat, Isopropylacrylat, n-Butylacrylat, Isobutylacrylat, tert.-Butylacrylat, n-Octylacrylat, iso-Octylacrylat, 2-Ethylhexylacrylat, Cyclopentylacrylat, Cyclohexylacrylat, Laurylacrylat, Palmitylacrylat und Stearylacrylat. Die Formel II umfaßt außerdem Hydroxyethylacrylat, Hydroxypropylacrylat und Hydroxybutylacrylat, wobei für diejenigen Hydroxyalkylacrylate, die sich von $C_3$- und $C_4$-Glykolen ableiten, sämtliche möglichen isomeren Glykole oder Mischungen davon in Betracht kommen, sowie Dimethylaminoethylacrylat, Diethylaminoethylacrylat, Dimethylaminopropylacrylat, Diethylaminopropylacrylat, Dimethylaminobutylacrylat, Diethylaminobutylacrylat, Dimethylaminoeopentylacrylat, Diethylaminoneopentylacrylat, sowie die neutralisierten bzw. quaternierten Dialkylaminoalkylacrylate. Als Quaternierungsmittel kommen beispielsweise Dimethylsulfat, Diethylsulfat, Methylchlorid, Ethylchlorid, Methylbromid, Ethylbromid und Benzylchlorid in Betracht. Die Salze der Dimethylaminoalkylacrylate werden durch Neutralisieren mit Salzsäure, Bromwasserstoffsäure, Schwefelsäure, Phosphorsäure oder gesättigten Carbonsäuren, wie

Ameisensäure und Essigsäure erhalten.
Verbindungen der Formel II mit

$$X = \underset{\overset{\|}{O}}{C}-OR$$

und $R = -(C_2H_4O)_pR^4$, wobei $R^4 = C_1$- bis $C_{18}$-Alkyl und p = 1 bis 80 sein kann, sind z. B. Methoxyethylacrylat, Ethoxyethylacrylat, Lauryloxi-tri-oxyethylacrylat, Methoxypolyoxyethylacrylate mit einem Gehalt an 1 bis 80, vorzugsweise 3 bis 30 Oxyethylgruppen.

Von der Verbindungen der Formel II werden vorzugsweise Acrylnitril, Methacrylat, Ethylacrylat, 2-Ethylhexylacrylat, Hydroxyethylacrylat und Hydroxybutylacrylat eingesetzt.

Acrylnitril und die genannten Acrylester werden in dem Temperaturbereich von 0 bis 150, vorzugsweise von 0 bis 70 °C in wäßrigem Medium bei einem pH-Wert von 7 bis 13, vorzugsweise 8-10 in Gegenwart von tertiären Aminen als Katalysator umgesetzt. Der pH-Wert des Reaktionsgemisches wird durch Zugabe der als Katalysator wirkenden tertiären Amine eingestellt. Sollte die wäßrige Formaldehydlösung einen sehr niedrigen pH-Wert haben, empfiehlt es sich, vor der Umsetzung mit Basen zu neutralisieren, z. B. mit Natronlauge, Kalilauge, Soda, Calciumhydroxid oder Bariumhydroxid. Der Formaldehyd wird vorzugsweise in Form einer wäßrigen Lösung, die .5 bis 40 Gew.% Formaldehyd enthält, oder als Halbacetal eingesetzt. Man kann jedoch auch Formaldehyd gasförmig mit den Verbindungen der Formel II in einem Reaktor umsetzen, jedoch ist es für erforderlich, daß die Reaktion dann in Gegenwart von Wasser erfolgt, damit eine Hydratisierung des Formaldehyds erfolgt. Vorzugweise verwendet man handelsübliche konzentrierte wäßrige Lösungen von Formaldehyd. Der Formaldehyd ist auch in Form einer alkoholischen Lösung oder als reines Halbacetal einsetzbar. Bevorzugt als Lösemittel und Halbacetalbildner eingesetzte Alkohole sind : Methanol, Ethanol, n- oder iso-Propanol, Butanole, Hexanol oder Cyclohexanol, Ethylenglykol, Propylenglykol, Methyl- oder Ethylglykol, Hexandiol.

Die Umsetzung wird vorzugsweise in homogener Mischung durchgeführt. Sofern man Acrylester verwendet, die sich in Wasser nur sehr schlecht lösen, empfliehlt es sich, die Umsetzung in Gegenwart eines gegenüber den Reaktionsteilnehmern inerten Lösemittels durchzuführen, das sowohl mit den Reaktionsteilnehmern und mit Wasser mischbar ist. Auf 100 Gewichtsteile der Mischung aus den Verbindungen der Formel II und Formaldehyd kann man bis zu 1000, vorzugsweise 10 bis 200 Gewichtsteile eines inerten Lösemittels einsetzen. Solche Lösemittel sind beispielsweise alle mit Wasser mischbaren Alkohole, z. B. Methanol, Ethanol, Isopropanol, n-Propanol, Butanol, Ethylenglykol, Propylenglykol, teilweise oder vollständig veretherte $C_2$- bis $C_4$-Glykole, Polyethylenglykole eines Molekulargewichts bis 800, Dioxan, Tetrahydrofuran und Acetonitril. In manchen Fällen mag es auch vorteilhaft sein, ein Gemisch mehrerer Lösemittel einzusetzen, z. B. eine Mischung aus Tetrahydrofuran und Methanol oder eine Mischung aus Acetonitril, Dioxan und Methanol.

Die Umsetzung erfolgt in Gegenwart von tertiären Aminen. Man kann sowohl offenkettige aliphatische Amine als auch cyclische tertiäre Amine verwenden. Beispiele hierfür sind : Trimethylamin, Triethylamin, Tri-n-propylaminm, Triisopropylamin, Tributylamin, Triisobutylamin, Tri-n-pentylamin, N-Methyldiisopropylamin, N,N-Diethylisopropylamin, N,N-Dimethylethylamin, N,N-Dimethylisopropylamin, Tri-2-ethylhexylamin, N-Methyldiethylamin, N,N-Dimethyl-n-propylamin, N,N-Dimethyl-n-butylamin, N,N-Dimethyl-isobutylamin, N,N-Dimethyl-(2-ethylhexyl) amin, N,N-Diisopropyl-(2-ethylhexyl)-amin, N,N-Di-n-butyl-(2-ethylhexyl)-amin, N-Methyl-di-(2-ethylhexyl) amin, N-n-butyl-di-(2-ethylhexyl) amin, N-Isobutyl-di(2-ethylhexyl)-amin, 1,4-Diazabicyclo-[2,2,2]-octan, Pyrrocolin und Chinolidin. Bezogen auf 100 Gewichtsteile der Mischung aus den Verbindungen der Formel II und Formaldehyd setzt man 0,1 bis 10, vorzugsweise 3 bis 8 Gewichtsteile eines tertiären Amins ein. Die Umsetzung wird bevorzugt in Gegenwart von 1,4-Diazabicyclo-[2,2,2]-octan, Pyrrocolin und Chinolidin durchgeführt. Die Verbindungen der Formel II und Formaldehyd werden in aller Regel in molarem Verhältnis miteinander zur Reaktion gebracht. Technische Gegebenheiten können es jedoch auch erforderlich machen, die eine oder die andere Komponente im Überschuß einzusetzen. So ist es beispielsweise möglich, das molare Verhältnis von Formaldehyd zu den Verbindungen der Formel II in dem Bereich von 20:1 bis 1:2, vorzugsweise 5:1 bis 0,75:1 zu variieren. Die Reaktion wird üblicherweise bei Atmosphärendruck durchgeführt. Sie kann jedoch auch bei vermindertem oder erhöhtem Druck vorgenommen werden. Ein Arbeiten unter Druck ist vor allem dann erforderlich, wenn die Umsetzung bei Temperaturen oberhalb von 100 °C vorgenommen wird.

Die nach dem erfindungsgemäßen Verfahren hergestellten Reaktionsprodukte-2-(1-Hydroxymethyl)-acrylnitril und -acrylester der oben angegebenen Formel I sind wertvolle Zwischenprodukte, die aufgrund ihrer Struktur vor allem als Monomere zur Herstellung von Polymerisaten verwendet werden.

## Beispiel 1

In einem Kolben werden 258 g (3 Mol) Methylacrylat, 225 g (3 Mol) 40 %iger wäßriger Formaldehyd und 20 g (0,17 Mol) 1,4-Diazabicyclo-[2,2,2]-octan und 250 ml Methanol gemischt. Der Kolben wird dann geschlossen und 48 Stunden bei Raumtemperatur auf einer Maschine geschüttelt. Der pH-Wert des Reaktionsgemisches beträgt ca. 9. Nach Beendigung der Reaktion gibt man zum Reaktionsgemisch

soviel konzentrierte Salzsäure hinzu, daß sich ein pH-Wert von 5,5 einstellt, setzt dann 400 ml Diethylether zu, schüttelt die Mischung kräftig durch und wartet anschließend, bis sich die Phasen getrennt haben. Die organische Phase wird abgetrennt, mit 100 ml einer gesättigten Kochsalzlösung gewaschen und über Natriumsulfat getrocknet. Anschließend wird das organische Lösemittel abdestilliert und der Rückstand einer fraktionierten Destillation unterworfen. Man erhält 261 g (75 % der Theorie) 2-(1-Hydroxymethyl)-methylacrylat in Form einer farblosen Flüssigkeit vom Siedepunkt von 72 bis 76 °C bei einem Druck von 0,3 mbar.

Die Umsetzung läßt sich in analoger Weise und mit ähnlich guten Ausbeuten mit Tetrahydrofuran anstelle von Methanol durchführen.

Beispiel 2

In einem Kolben werden 159 g (3 Mol) Acrylnitril, 225 g (3 Mol) einer 40 %igen wäßrigen Lösung von Formaldehyd und 12,5 g (0,11 Mol), 1,4-Diazabicyclo-[2,2,2]-octan gemischt und 48 Stunden bei Raumtemperatur gerührt. Der pH-Wert bei der Umsetzung beträgt ca. 9. Nach Beendigung der Umsetzung werden die leicht flüchtigen Anteile bei einem Druck von 14 mbar abdestilliert. Der Rückstand wird in 400 ml Diethylether gelöst. Die etherische Lösung wird dann mit 70 ml einer 8 %igen wäßrigen Salzsäure und anschließend mit 70 ml einer gesättigten wäßrigen Kochsalzlösung gewaschen und über Natriumsulfat getrocknet. Nach dem Abdestillieren des Ethers fraktioniert man den Rückstand bei einem Druck von 0,3 mbar. Bei diesem Druck erhält man im Temperaturbereich von 84 bis 86 °C 164 g (66 % der Theorie) 2-(1-Hydroxymethyl)-acrylnitril in Form eines farblosen Produkts.

Beispiel 3

184 g (1 Mol) 2-Ethylhexylacrylat, 75 g (1 Mol) 40 %iger wäßriger Formaldehyd und 11,4 g (0,1 Mol) 1,4-Diazabicyclo [2.2.2.] octan (im folgenden Text mit DABCO abgekürzt) werden nach Zusatz von 100 ml Ethanol 36 Stunden auf 50 °C erhitzt. Der pH-Wert des Reaktionsgemisches beträgt zu Reaktionsbeginn 9,7.

Nach Reaktionsende wird mit konzentrierter Salzsäure ein pH-Wert von 5 eingestellt und mit 200 ml Diethylether extrahiert.

Die organische Phase wird mit 50 ml Wasser gewaschen und über Natriumsulfat getrocknet. Nach dem Abdestillieren des organischen Lösemittels und anderer leicht flüchtiger Anteile verbleiben 152 g (71 % der Theorie) 2-(1-Hydroxymethyl) 2′-ethylhexylacrylat als farblose Flüssigkeit.

Beispiel 4

26 g (0,2 Mol) Hydroxypropylacrylat, 15 g (0,2 Mol) Formalin (42,5 %ig), 2 g (17,8 mmol) DABCO und 20 g Ethanol werden 72 Stunden bei Raumtemperatur intensiv gerührt.

Anschließend stellt man mit halbkonzentrierter Salzsäure einen pH-Wert von 5 ein und schüttelt 3 mal mit je 40 ml Dichlormethan aus.

Nach Waschen der vereinigten organischen Phasen mit 30 ml Wasser und Trocknen über Natriumsulfat werden Lösemittel und andere flüchtige Anteile im Vakuum entfernt.

Es verbleiben 26 g einer schwach gelben Flüssigkeit 2-(1-Hydroxymethyl)-3′-hydroxypropylacrylat (81 % der Theorie).

Beispiel 5

Es werden 68,4 g (0,4 mol) N,N-Diethylaminoethylacrylat, 32 g (0,4 mol) Formalin (40 %ig) und 4,0 g (35,0 mmol) DABCO 50 Stunden bei 25 °C intensiv gerührt.

Anschließend wird mit halbkonzentrierter Salzsäure ein pH-Wert von 6 eingestellt und 3 mal mit je 50 ml Dichlormethan ausgeschüttelt. Nach dem Waschen mit 50 ml einer gesättigten Kochsalzlösung und Trocknen der organischen Phase über Natriumsulfat, werden in Vakuum flüchtige Anteile entfernt.

Es verbleiben 61 g 2(1-Hydroxymethyl)-2′-diethylaminoethylacrylat (75 % der Theorie).

Beispiel 6

Eine Lösung von 11 g (0,36 mol) wasserfreiem, monomerem Formaldehyd in 100 ml absolutem Ethanol wird mit 43 g (0,5 mol) Acrylsäuremethylester und 3 g (26,7 mmol) DABCO versetzt und 72 Stunden bei Raumtemperatur stehengelassen. Anschließend werden flüchtige Anteile im Wasserstrahlvakuum entfernt. Den Rückstand nimmt man in 100 ml Diethylether auf und wächst die etherische Lösung mit 20 ml einer 10 %igen wäßrigen Salzsäure sowie mit 30 ml einer gesättigten wäßrigen Kochsalzlösung. Nach dem Trocknen der organischen Phase über Natriumsulfat und dem Entfernen des Lösemittels im Wasserstrahlvakuum wird wie im Beispiel 1 ausgeführt fraktionierend destilliert. Es resultieren 25 g (59 % der Theorie) 2-(1-Hydroxymethyl) methylacrylat.

## Beispiel 7

In einem Kolben werden 86 g (1 Mol) Methylacrylat, 75 g (1 Mol) 40 %iger Formaldehyd und 7 g (0,06 mol) DABCO gemischt. Anschließend wird 72 Stunden intensiv gerührt.

Die Aufarbeitung erfolgt analog zu der unter Beispiel 1 beschriebenen. Ausbeute : 48 g (41 % der Theorie) 2(1-Hydroxymethyl)-methylacrylat.

Bei analoger Versuchsführung mit Triethylamin anstelle von DABCO wird eine Ausbeute von 31 % der Theorie erhalten.

## Beispiel 8

In einem Kolben werden 100 g (1 Mol) Ethylacrylat, 113 g (1,5 Mol) 40 %iger wäßriger Formaldehyd und 7 g (62 mmol) DABCO und 100 ml Acetonitril gemischt. Der pH-Wert des Reaktionsgemisches beträgt zu Beginn 8,9.

Nach 40-stündigem intensivem Rühren bei 50 °C gibt man zum Reaktionsgemisch soviel halbkonzentrierte Salzsäure hinzu, daß sich ein pH-Wert von 5,0 einstellt, setzt dann 200 ml Diethylether zu, schüttelt kräftig durch und trennt die organische Phase ab. Diese wird mit 50 ml einer gesättigten, wäßrigen Kochsalzlösung gewaschen und über Natriumsulfat getrocknet. Nach Abdestillieren des organischen Lösemittels wird der Rückstand einer fraktionierten Destillation unterworfen.

Man erhält 86 g (66 % der Theorie) 2(1-Hydroxymethyl)-ethylacrylat vom Siedepunkt von 84-86 °C bei einem Druck von 0,4 mbar.

## Beispiel 9

Die Umsetzung von 130 g (1 mol) Cyclohexanol-Formaldehyd-Halbacetal in 100 g Cyclohexanol und 86 g (1 mol) Methylacrylat führt nach Zugabe von 8 g (71,4 mmol) 1,4-Diazabicyclo [2.2.2] octan nach 70-stündigem Rühren bei 25 °C zu einer 45 %igen Bildung von 2(1-Hydroxymethyl) methylacrylat [HPLC-Auswertung].

**Patentansprüche**

1. Verfahren zur Herstellung von 2-(1-Hydroxymethyl)-acrylnitril und -acrylestern der Formel

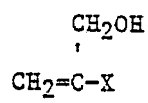

(I)

in der X =

$-CN$, 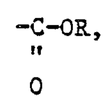

R = $C_1$- bis $C_{18}$-Alkyl, $-(CH_2)_n-OH$,

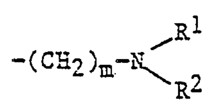 ,

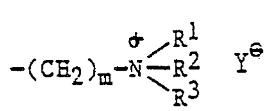 ,

$-(C_2H_4O)_pR^4$

$R^1$, $R^2$ = $-CH_3$, $-C_2H_5$

$R^3$ = $-H$, $-CH_3$, $-C_2H_5$,

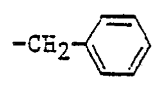

$R^4$ = $C_1$- bis $C_{18}$-Alkyl

n = 2 bis 4,
m = 2 bis 5,
p = 1 bis 80 und
$Y^- = Cl^-$, $Br^-$, $SO_4^{2-}$, $PO_4^{3-}$, $CH_3OSO_3^-$, $C_2H_5OSO_3^-$, $CH_3COO^-$, $HCOO^-$
bedeutet, dadurch gekennzeichnet, daß man eine Verbindung der Formel

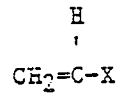
(II)

in der X die in Formel I angegebene Bedeutung hat, mit hydratisiertem Formaldehyd oder Halbacetalen des Formaldehyds, die sich von $C_1$- bis $C_6$-Alkoholen ableiten, in Gegenwart von tertiären Aminen als Katalysator umsetzt.

2. Verfahren nach Anspruch 1, dadurch gekennzeichnet, daß man die Umsetzung bei einer Temperatur von 0 bis 150 °C durchfürht.

3. Verfahren nach den Ansprüchen 1 und 2, dadurch gekennzeichnet, daß man die Umsetzung in Gegenwart von Wasser oder Alkoholen, bei einem pH-Wert von 8 bis 10 durchführt.

4. Verfahren nach den Ansprüchen 1 bis 3, dadurch gekennzeichnet, daß man die Umsetzung in Gegenwart eines inerten Lösemittels durchführt, das mit den Reaktionsteilnehmern und mit Wasser mischbar ist.

5. Verfahren nach den Ansprüchen 1 bis 3, dadurch gekennzeichnet, daß man auf 100 Gewichtsteile der Mischung aus den Verbindungen der Formel II und Formaldehyd und/oder den Halbacetalen des Formaldehyds, 10 bis 200 Gewichtsteile eines $C_1$- bis $C_4$-Alkohols, Dioxan, Tetrahydrofuran, Dimethylformamid und/oder Acetonitril einsetzt.

6. Verfahren nach den Ansprüchen 1 bis 5, dadurch gekennzeichnet, daß man, bezogen auf 100 Gewichtsteile der Mischung aus den Verbindungen der Formel II und hydratisiertem Formaldehyd bzw. Halbacetalen des Formaldehyds 0,1 bis 10 Gewichtsteile eines tertiären Amins einsetzt.

7. Verfahren nach Anspruch 6, dadurch gekennzeichnet, daß man als tertiäre Amine 1,4-Diazabicyclo-[2,2,2]-octan, Pyrrocolin und Chinolidin einsetzt.

8. Verfahren nach dem Ansprüchen 1 und 2, dadurch gekennzeichnet, daß man das Halbacetal des Formaldehyds mit Methanol einsetzt.

**Claims**

1. A process for the preparation of 2-(hydroxymethyl)-acrylonitrile and 2-(hydroxymethyl)-acrylates of the formula

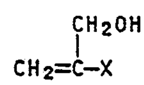
(I)

where X is —CN or

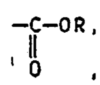

R is $C_1$-$C_{18}$-alkyl,

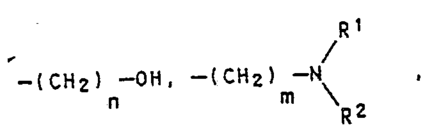

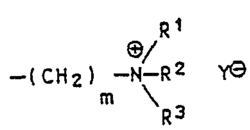

or $-(C_2H_4O)_pR^4$
$R^1$ and $R^2$ are each $-CH_3$ or $-C_2H_5$,
$R^3$ is —H, $-CH_3$, $-C_2H_5$ or

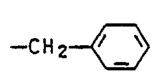

$R^4$ is $C_1$-$C_{18}$-alkyl,
n is from 2 to 4,
m is from 2 to 5,
p is from 1 to 80, and
$Y^\ominus$ is $Cl^-$, $Br^-$, $SO_4^{2-}$, $PO_4^{3-}$, $CH_3OSO_3^-$, $C_2H_5OSO_3^-$, $CH_3COO^-$ or $HCOO^-$,
wherein a compound of the formula

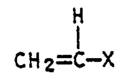

(II)

where X has the meanings stated in formula I, is reacted with hydrated formaldehyde or a formaldehyde hemiacetal which is derived from a $C_1$-$C_6$-alcohol, in the presence of a tertiary amine as catalyst.

2. A process as claimed in claim 1, wherein the reaction is carried out at from 0 to 150 °C.

3. A process as claimed in claims 1 and 2, wherein the reaction is carried out in the presence of water or an alcohol at a pH of from 8 to 10.

4. A process as claimed in claims 1 to 3, wherein the reaction is carried out in the presence of an inert solvent which is miscible with the reactants and with water.

5. A process as claimed in claims 1 to 3, wherein from 10 to 200 parts by weight of a $C_1$-$C_4$-alcohol, dioxane, tetrahydrofuran, dimethylformamide and/or acetonitrile are used per 100 parts by weight of the mixture of the compound of the formula II and formaldehyde and/or the formaldehyde hemiacetal.

6. A process as claimed in claims 1 to 5, wherein from 0.1 to 10 parts by weight of a tertiary amine are used per 100 parts by weight of the mixture of the compound of the formula II and hydrated formaldehyde or a formaldehyde hemiacetal.

7. A process as claimed in claim 6, wherein the tertiary amine used is 1,4-diazabicyclo [2.2.2] octane, pyrrocoline or quinolidine.

8. A process as claimed in claims 1 and 2, wherein the hemiacetal of formaldehyde with methanol is used.

**Revendications**

1. Procédé de préparation de 2-(1-hydroxymethyl)-acrylonitryle et -acryloesters de formule

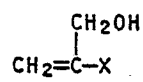

(I)

dans laquelle
X = —CN,

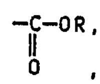

R = -Alkyle en $C_1$- à $C_{18}$, $-(CH_2)_n-OH$,

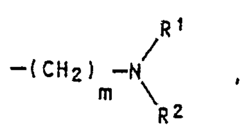

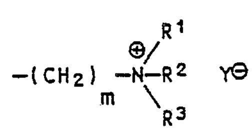

$-(C_2H_4O)_pR^4$
$R^1$, $R^2$ = $-CH_3$, $-C_2H_5$
$R^3$ = —H, $-CH_3$, $-C_2H_5$,

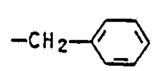

$R^4$ = -Alkyle en $C_1$- à $C_{18}$
n = 2 à 4,
m = 2 à 5,
p =1 à 80 et
$Y^-$ = $Cl^-$, $Br^-$, $SO_4^{2-}$, $PO_4^{3-}$, $CH_3OSO_3^-$, $C_2H_5OSO_3^-$, $CH_3COO^-$, $HCOO^-$
caractérisé par le fait que l'on fait réagir, en présence d'amines tertiaires, comme catalyseur, un composé de formule

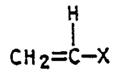

(II)

dans laquelle X a la signification donnée dans la formule I, avec du formaldéhyde hydraté ou des semi-acétals de formaldéhyde, qui dérivent d'alcools en $C_1$ à $C_6$.

2. Procédé selon la revendication 1, caractérisé par le fait que l'on effectue la réaction à une température de 0 à 150 °C.

3. Procédé selon la revendication 1 et 2, caractérisé par le fait que l'on effectue la réaction en présence d'eau ou d'alcools, à un pH de 8 à 10.

4. Procédé selon les revendications 1 à 3, caractérisé par le fait qu'on effectue la réaction en présence d'un solvant inerte qui est miscible avec les participants de la réaction et avec l'eau.

5. Procédé selon la revendication 1 à 3, caractérisé par le fait que, sur 100 parties en poids du mélange des composés de formule II et de formaldéhyde et/ou semi-acétals de formaldéhyde, on introduit 10 à 200 parties en poids d'un alcool en $C_1$ à $C_4$, de dioxane, tétrahydrofurane, diméthylformamide et/ou acétonitrile.

6. Procédé selon les revendications 1 à 5, caractérisé par le fait que, sur 100 parties en poids du mélange des composés de formule II et de formaldéhyde hydraté ou semi-acétals de formaldéhyde, on introduit 0,1 à 10 parties en poids d'une amine tertiaire.

7. Procédé selon la revendication 6, caractérisé par le fait que l'on introduit, comme amine tertiaire, 1,4-diazabicyclo-[2,2,2]-octane, pyrrocoline et quinolidine.

8. Procédé selon les revendications 1 et 2, caractérisé par le fait que l'on introduit le semi-acétal de formaldéhyde avec du méthanol.